# EUROPEAN PATENT APPLICATION

(11) **EP 4 659 717 A2**
(43) Date of publication of application: **10.12.2025**
(21) Application number: 25209506.2
(22) Date of filing: 11.07.2023
(51) Int. Cl.: A61F 2/95

(54) **FRICTION PAD FOR DEPLOYMENT AND RESHEATHING OF INTRAVASCULAR IMPLANT**

(30) Priority: 12.08.2022 US 202263397679 P
(62) Divisional of application: 23751232.2
(71) Applicant: Stryker European Operations Limited, Carrigtwohill, Co. Cork T45 HX08 (IE); Stryker Corporation, Portage, MI 49002 (US)
(72) Inventor: LOGANATHAN, Siddharth, Santa Clara, 95050 (US); LOVELACE, Brad, Fremont, 94538 (US); MARLEY, Allison, San Jose, 95131 (US); BROCHU, Alice, Livermore, 94550 (US)
(74) Representative: Gillespie, Richard

(57) **Abstract**

An intravascular implant delivery system comprises an intravascular implant having a tubular implant body and a central implant lumen. The system further comprises a delivery catheter having an elongate sheath body and an inner sheath lumen. The intravascular implant is disposed within the inner sheath lumen when in a compressed delivery configuration. The system further comprises a pusher member assembly slidably disposed in the inner sheath lumen. The pusher member assembly comprises an elongate pusher member and a friction pad having a radially compressed state and a radially expanded state. The friction pad is disposed within the central implant lumen when in the radially compressed state. The friction pad comprises a tubular pad body and a central pad lumen in which the pusher member is disposed. The tubular pad body has an elastomeric base material and modulating elements embedded within and axially extending along the elastomeric base material.

## Description

### Field

The present disclosure relates generally to minimally invasive assemblies used for delivering medial implants, and more particularly, to delivery assemblies for delivering medical implants, such as a tubular stent or flow diverter, to a targeted implantation site in the vasculature of a patient.

### Background

The use of intravascular implants, such as stents, stent grafts, flow-diverters, vaso-occlusive devices, vena cava filters, etc., has become an effective method for treating many types of vascular disease. In general, a suitable intravascular implant is inserted into the vascular system of the patient and navigated through the vasculature to a desired target site using a delivery system. Using currently available delivery systems, virtually any target site in the patient's vascular system may be accessed, including the coronary, cerebral, and peripheral vasculature.

In a typical intravascular medical device delivery procedure, a delivery catheter is percutaneously introduced into the vasculature of the patient over a guidewire and/or through a guide sheath. The open distal end of the delivery catheter is then navigated to a targeted implantation site using well-known techniques. An intravascular implant is delivered through a lumen of the delivery catheter in a compressed (i.e., reduced diameter) delivery configuration. For example, a pusher member assembly having a pusher member (e.g., a core wire) on which the intravascular implant is temporarily affixed may be used to push the intravascular implant through the lumen of the delivery catheter. When the intravascular implant is located at the adjacent the desired location in the vasculature, the intravascular implant can then be deployed out of the open distal end of the delivery catheter (e.g., by withdrawing the delivery catheter relative to the intravascular implant) and placed within an expanded (i.e., increased diameter) deployed configuration into engagement with the interior wall of the blood vessel. A radiopaque marker may be located at the distal tip of the delivery catheter, so that the implantation site of the intravascular implant can be estimated with the assistance of medical imaging technology (e.g., fluoroscopy).

In one intravascular implant delivery method, the intravascular implant may be mounted over a balloon, which can then be inflated to radially expand the intravascular implant outward into engagement with the interior wall of the blood vessel. In another intravascular implant delivery method, the intravascular implant may be self-expanding and loaded into the lumen of the catheter in an elastically compressed state, such that the intravascular implant elastically expands once deployed out of the open distal end of the catheter without requiring assistance from a balloon.

Oftentimes, once the intravascular implant is at least partially deployed from the delivery catheter, a physician may determine that the actual implantation site of the intravascular implant may be located away from the targeted implantation site of the intravascular implant, and thus, clinically undesirable. The actual implantation site of the intravascular implant may be clinically undesirable for a number of reasons. For example, if the intravascular implant is a stent for bridging the neck of an aneurysm or diverting flow from an aneurysm or a blood vessel, an accurate implantation of the stent at the targeted implantation site is needed. If the stent fails to cover the entire aneurysm, or the stent is located on a bend that may cause a thrombosis or stroke, or the stent covers another vessel, the intravascular stent may be need to be repositioned. In the case of a self-expanding intravascular implant, if the intravascular implant has not been deployed from the delivery catheter past a point-of-no-return (i.e., the point at which the intravascular implant cannot be retracted back into the delivery catheter), the intravascular stent may be resheathed within the delivery catheter and repositioned, such that the intravascular implant can be redeployed at the targeted implantation site.

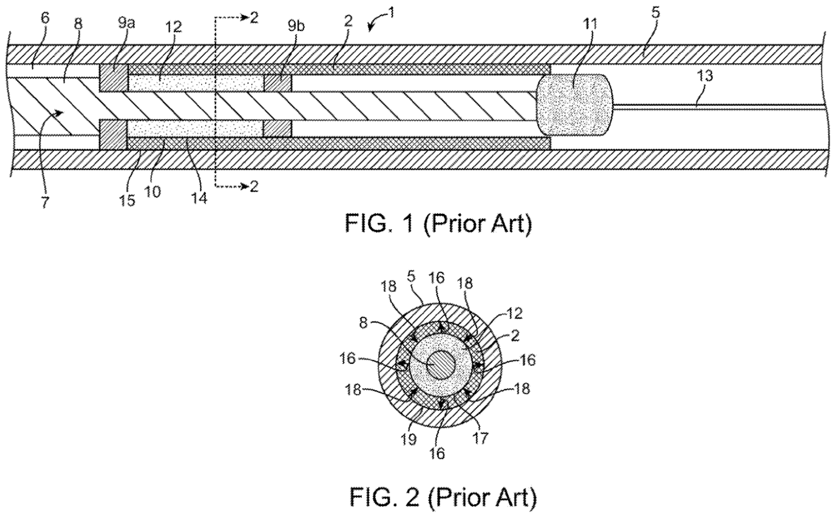

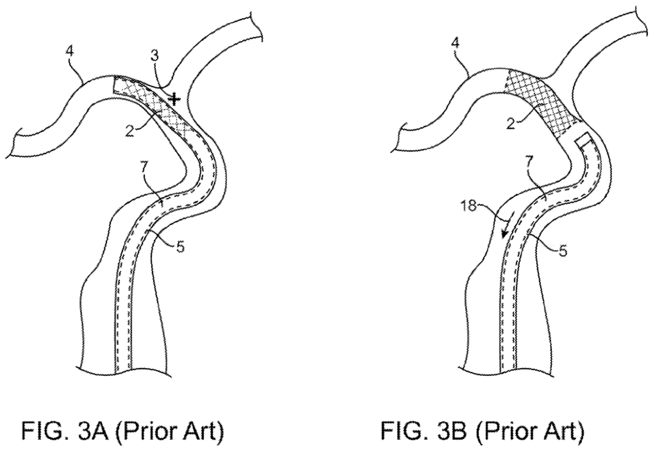

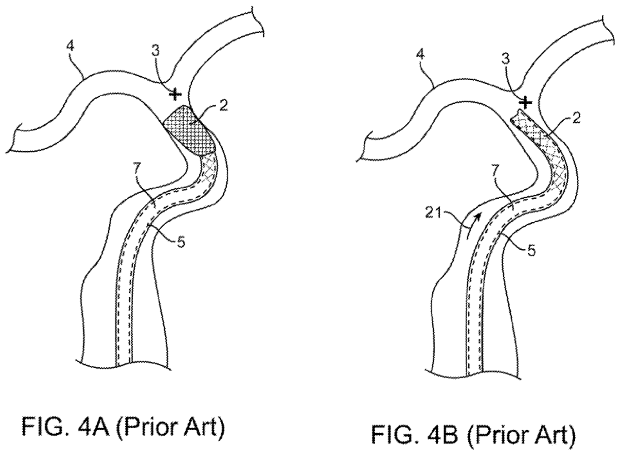

Referring to **Figs. 1-2****,** one embodiment of a delivery system 1 for delivering an intravascular implant 2 at a targeted implantation site 3 within a blood vessel 4 (shown in **Figs. 3A-3B** and **4A-4B).** The delivery system 1 comprises a delivery catheter 5 having an inner lumen 6, and a pusher member assembly 7 disposed within the inner lumen 6 of the deliver sheath 5. The pusher member assembly 7 comprises a pusher member 8 and a proximal bumper 9a and a distal bumper 9b affixed to the pusher member 8, thereby forming an annular space 10 between the bumpers 9a, 9b. The proximal bumper 9a may serve as a radiopaque marker to indicate the proximal end of the intravascular implant 2, so that full deployment of the intravascular implant 2 from the delivery catheter 5 can be confirmed. The distal bumper 9b may serve as a radiopaque marker that indicates the point-of-no-return as the intravascular implant 2 is being deployed from the delivery catheter 5, after which the intravascular implant 2 may no longer be resheathed into the delivery catheter 5. Other radiopaque markers (not shown) may be located on the distal end of the delivery catheter 5 and other locations along the pusher member 8. The pusher member assembly 7 may further comprise a protective device 11 located at the distal end of the intravascular implant 2. The pusher member assembly 7 further comprises an elastomeric friction pad 12 disposed on the pusher member 8 within the annular space 10 between the bumpers 9a, 9b. The proximal and distal bumpers 9a, 9b serve to retain the friction pad 12 therebetween. The pusher member assembly 7 further comprises an atraumatic distal portion 13 affixed to the distal end of the pusher member 8.

The intravascular implant 2 is disposed within the inner lumen 6 of the delivery catheter 5 between the outer surface 14 of the friction pad 12 and the inner surface 15 of the delivery catheter 5. The friction pad 12 is disposed within the inner lumen 6 of the delivery catheter 5 in a compressed state, such that friction pad 12 imparts a radially outward force 16 against an inner surface 17 of the intravascular implant 2, while the inner lumen 6 of the delivery catheter 5 imparts a radially inward force 18 against an outer surface 19 of the intravascular implant 2, as best illustrated in **Fig. 2****.** In this manner, the intravascular implant 2 is frictionally engaged with the pusher member assembly 7, such that the intravascular implant 2 moves in unison with the pusher member assembly 7 as the pusher member assembly 7 and delivery catheter 5 are axially translated relative to each other (e.g., by translating the delivery catheter 5 in the proximal direction 18 while holding the pusher member assembly 7 in place) to deploy the intravascular implant 2 out of a distal port 19 of the delivery catheter 5 and into the blood vessel 4, as illustrated in **Figs. 3A** and **3B****.**

Because the tensile strength of the intravascular implant 2, alone, is very low, the friction pad 12 not only facilitates deployment of the intravascular implant 2, it serves to facilitate resheathing of the intravascular implant 2 within the inner lumen 6 of the delivery catheter 5 (e.g., by translating the delivery catheter 5 in the distal direction 21 while holding the pusher member assembly 7 in place), as illustrated in **Figs. 4A** and **4B****.** As long as the intravascular implant 2 has not been deployed from the delivery catheter 5 past the point-of-no-return (i.e., as long as distal bumper 9b remains in the inner lumen 6 of the delivery catheter 5, indicating that the friction pad 12, which will tend to expand outside of the constraints of the delivery catheter 5, is fully within the inner lumen 6 of the delivery catheter 5), the intravascular implant 2, facilitated by the friction pad 12, may be resheathed back into the delivery catheter 5.

The performance characteristics of the friction pad 12 should be selected in a manner that the intravascular implant 2 is frictionally engaged to enable both deployment and resheathing functionality of the intravascular implant 2. For example, the friction pad 12 should have low lateral bending stiffness paired with a low radial strength (high radial compressibility/expandability) that can be tailored or calibrated, such that that the friction pad 12 engages the intravascular implant 2, while avoiding high track/resheath force that may otherwise prevent deployment, resheathing, or redeployment of the intravascular implant 2 without damaging the intravascular implant 2 or generate particles from the ends of the intravascular implant 2. In particular, to frictionally engage the intravascular implant 2 without excessively creating a high track/resheath force, it is desirable that enough outward radial force 16 be applied by the friction pad 12 to the inner surface 17 of the intravascular implant 2, but not so much outward radial force that creates excessive frictional force between the outer surface of the intravascular implant 2 and the inner surface of the delivery catheter 5 that may otherwise confound translation of the intravascular implant 2 within the inner lumen 6 of the delivery catheter 5 and/or resheathing of the intravascular implant 2 back into the inner lumen 6 of the delivery catheter 5. Furthermore, it is desirable that the lateral flexibility of the friction pad 12 be low enough, so that the friction pad 12 easily conforms to the bend in the vasculature of the patient, such that the friction pad 12 more easily tracks through the bend in the vasculature of the patient, thereby facilitating axial translation between the pusher member assembly 7 and the delivery catheter 5 as the friction pad 12 traverses the bend in the vasculature of the patient. Traditionally an elastomeric friction pad having a low durometer (or modulus of elasticity) will provide the friction pad with such sufficiently low bending stiffness and low radial strength.

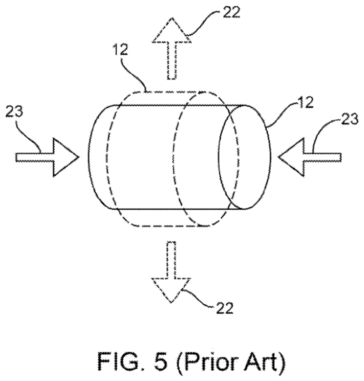

However, as illustrated in **Fig. 5****,** an elastomeric friction pad 12 composed of a low durometer material has a relatively low columnar strength, such that it is prone to radial outward bulging (shown in phantom) in the radial direction 22 in response to an axially compressive force 24, e.g., caused by increased resistance to the translation of the intravascular implant 2 within the inner lumen 6 of the delivery catheter 5 during deployment and/or resheathing of the intravascular implant 2. This radial outward bulging of the friction pad 12 can prohibitively increase track/resheath force of the intravascular implant 2, while also increasing the potential for particle generation at the ends of the intravascular implant 2. In some cases, the increased track/resheath force may even cause the friction pad 12 to prolapse over the proximal bumper 9a or distal bumper 9b, thereby rendering the delivery system 1 unusable.

There, thus, remains an ongoing need to minimize radial outward bulging of a friction pad in the presence of an increased resistance to the translation of an intravascular implant within the lumen of a delivery catheter during deployment and/or resheathing of the intravascular implant, while also maintaining the desired radial compressibility/expandability and lateral flexibility of the friction pad.

### Summary

In accordance with the present inventions, an intravascular implant delivery system comprises an intravascular implant (e.g., one of stent, a stent graft, a flow-diverter, a vaso-occlusive device, and a vena cava filter) having a compressed delivery configuration and an expanded deployed configuration. The intravascular implant comprises a tubular implant body and a central implant lumen axially extending through the tubular implant body. The intravascular implant delivery system further comprises a delivery catheter having an elongate sheath body and an inner sheath lumen axially extending through the sheath body. The intravascular implant is disposed within the inner sheath lumen when in the compressed delivery configuration. The intravascular implant delivery system further comprises a pusher member assembly slidably disposed in the inner sheath lumen. The pusher member assembly comprises an elongate pusher member (e.g.., a delivery wire) and a friction pad having a radially compressed state and a radially expanded state. The friction pad is disposed within the central implant lumen when in the radially compressed state, and comprises a tubular pad body and a central pad lumen axially extending through the tubular pad body. The pusher member is disposed within the central pad lumen.

In one embodiment, the pusher member assembly further comprises a proximal bumper and a distal bumper affixed to the pusher member, thereby forming an annular space therebetween, wherein the friction pad is disposed within the annular space. In another embodiment, the pusher member assembly and delivery catheter can be axially translated relative to each other, and the friction pad is configured for frictionally engaging the intravascular implant as the pusher member assembly and delivery catheter are axially translated relative to each other, such that the intravascular implant and the pusher member assembly axially translate with each other in unison to deploy the intravascular implant from the inner sheath lumen into its expanded deployed configuration. In this embodiment, the friction pad may be configured for frictionally engaging the intravascular implant as the pusher member assembly and the delivery catheter are axially translated relative to each other, such that the intravascular implant and the pusher member assembly axially translate with each other in unison to resheath the intravascular implant, while at least in a partially expanded deployed configuration, back into its compressed delivery configuration within the inner sheath lumen. The tubular pad body has an elastomeric base material that forms the tubular pad body and at least one modulating element embedded within and axially extending along the elastomeric base material. In one embodiment, a plurality of modulating elements is circumferentially arranged within the elastomeric base material. In another embodiment, the tubular pad body is cylindrical. In still embodiment, the elastomeric base material of the friction pad is polyether block amide (PEBA). In yet another embodiment, each of the modulating element(s) are elongated. In this case, each of the elongated modulating element(s) may extend parallel to a longitudinal axis of the tubular pad body.

In accordance with a first aspect of the present inventions, each of the modulating element(s) has different mechanical properties than mechanical properties of the elastomeric base material. In one embodiment, the modulating element(s) increases a ratio of a columnar strength over a radial strength of the tubular pad body. As one example, each of the modulating element(s) may have a modulus of elasticity greater than a modulus of elasticity of the elastomeric base material, and may increase the columnar strength of the tubular pad body. In this case, each of the modulating element(s) may be a solid beam, and may have a durometer in the range of 25A-35D. As another example, each of the modulating element(s) may have a modulus of elasticity less than a modulus of elasticity of the elastomeric base material, and may decrease a radial strength of the friction pad. In this case, each of the modulating element(s) may be gel or air, and may have a durometer greater than 35D.

In accordance with a second aspect of the present inventions, the modulating element(s) increases a ratio of a columnar strength over a radial strength of the tubular pad body. As one example, each of the modulating element(s) may have a modulus of elasticity greater than a modulus of elasticity of the elastomeric base material, and may increase the columnar strength of the tubular pad body. In this case, each of the modulating element(s) may be a solid beam, and may have a durometer in the range of 25A-35D. As another example, each of the modulating element(s) may have a modulus of elasticity less than a modulus of elasticity of the elastomeric base material, and may decrease a radial strength of the friction pad. In this case, each of the modulating element(s) may be gel or air, and may have a durometer greater than 35D.

In accordance with a third aspect of the present inventions, the modulating element(s) increases a columnar strength of the tubular pad body. For example, each of the modulating element(s) may have a modulus of elasticity greater than a modulus of elasticity of the elastomeric base material. In this case, each of the modulating element(s) may be a solid beam, and may have a durometer in the range of 25A-35D.

In accordance with a fourth aspect of the present inventions, the modulating element(s) decreases a radial strength of the tubular pad body. For example, each of the modulating element(s) may have a modulus of elasticity less than a modulus of elasticity of the elastomeric base material. In this case, each of the modulating element(s) may be gel or air, and may have a durometer greater than 35D.

Other and further aspects and features of embodiments of the disclosed inventions will become apparent from the ensuing detailed description in view of the accompanying figures.

### Brief Description of the Drawings

The drawings illustrate the design and utility of preferred embodiments of the present inventions, in which similar elements are referred to by common reference numerals. It should be noted that the figures are not drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention, which is defined only by the appended claims and their equivalents. In addition, an illustrated embodiment of the disclosed inventions needs not have all the aspects or advantages shown. An aspect or an advantage described in conjunction with a particular embodiment of the disclosed inventions is not necessarily limited to that embodiment and can be practiced in any other embodiments even if not so illustrated. In order to better appreciate how the above-recited and the other advantages and objects of the present inventions are obtained, a more particular description of the present inventions briefly described above will be rendered by reference to specific embodiments thereof, which are illustrated in the accompanying drawings. Understanding that these drawings depict only typical embodiments of the invention and are not therefore to be considered limiting of its scope, the invention will be described and explained with additional specificity and detail through the use of the accompanying drawings in which:
**Fig. 1** is a longitudinal-sectional view of a prior art delivery system for delivering an intravascular implant into a patient.
**Fig. 2** is a cross-sectional view of the delivery system of **Fig. 1****,** taken along the **line 2-2.**
**Figs. 3A-3B** are plan views illustrating a prior art method using the delivery system of **Fig. 1** to deploy an intravascular implant within the vasculature of a patient.
**Figs. 4A-4B** are plan views illustrating a prior art method using the delivery system of **Fig. 1** to resheath the intravascular implant.
**Fig. 5** is a perspective view illustrating an elastomeric friction pad of the prior art delivery system, particularly showing the friction pad radially bulging in response to a compressive force.
**Fig. 6** is a plan view of one embodiment of an intravascular implant delivery system constructed in accordance with the present inventions.
**Fig. 7A** is a plan view of the distal end of the intravascular implant delivery system of **Fig. 6****.**
**Fig. 7B** is a cut-away plan view of the distal end of a pusher member assembly of the intravascular implant delivery system of **Fig. 7A****.**
**Fig. 7C** is a cut-away perspective view of the pusher member assembly of **Fig. 7B****.**
**Fig. 7D** is a view of an intravascular implant carried by the pusher member assembly of **Fig. 7B****.**
**Fig. 7E** is a close-up plan view of the distal tip of the pusher member assembly of **Fig. 7B****.**
**Fig. 7F** is a close-up plan view of a portion of the intravascular implant delivery system of **Fig. 7A****,** taken along the **line 7F-7F.**
**Fig. 7G** is a close-up plan view of the distal tip of the intravascular implant delivery system of **Fig. 7A****,** particularly showing the intravascular implant while deployed.
**Fig. 7H** is a cross-sectional view of the intravascular implant delivery system of **Fig. 7F****,** taken along the line **7H-7H.**
**Fig. 7I** is a cross-sectional view of the intravascular implant delivery system of **Fig. 7G****,** taken along the **line 7I-7I.**
**Fig. 8A** is a perspective view of a friction pad of the pusher member assembly of **Fig. 7B****,** particularly showing a reduction in the diameter of the friction pad in response to a radially compressive force.
**Fig. 8B** is a perspective view of the friction pad of **Fig. 8A****,** particularly showing no increase in the diameter of the friction pad in response to an axially compressive force.
**Fig. 9** is a perspective view of one embodiment of the friction pad of **Fig. 8A****.**
**Fig. 9A** is a cross-sectional view of the friction pad of **Fig. 9****,** taken along the **line 9A-9A.**
**Fig. 10** is a perspective view of one embodiment of the friction pad of **Fig. 8A****.**
**Fig. 10A** is a cross-sectional view of the friction pad of **Fig. 10****,** taken along the **line 10A-10A.**
**Fig. 11** is a perspective view of one embodiment of the friction pad of **Fig. 8A****.**
**Fig. 11A** is a cross-sectional view of the friction pad of **Fig. 11****,** taken along the **line 11A-11A.**
**Fig. 12** is a perspective view of one embodiment of the friction pad of **Fig. 8A****.**
**Fig. 12A** is a cross-sectional view of the friction pad of **Fig. 12****,** taken along the **line 12A-12A.**
**Fig. 13** is a perspective view of one embodiment of the friction pad of **Fig. 8A****.**
**Fig. 13A** is a cross-sectional view of the friction pad of **Fig. 13****,** taken along the **line 13A-13A.**
**Fig. 13B** is a cross-sectional view of the friction pad of **Fig. 13****,** taken along the **line 13B-13B.**

### Detailed Description of the Illustrated Embodiments

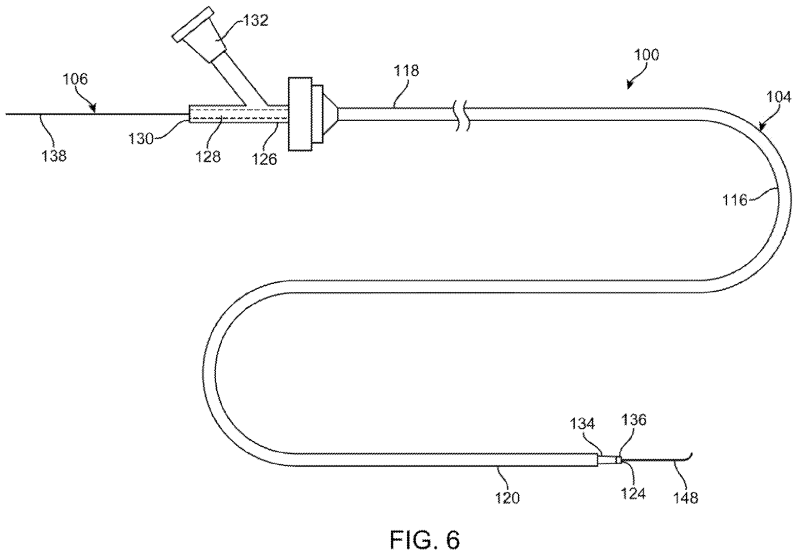

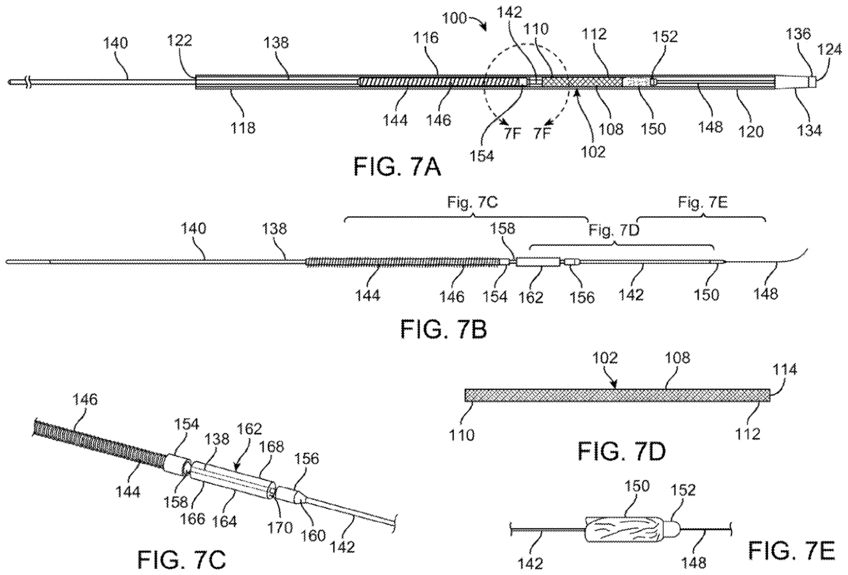

Referring to **Figs. 6** and **7A****-7I,** one embodiment of an implant delivery system 100 constructed in accordance with one embodiment of the present inventions will be described. The implant delivery system 100 generally comprises an intravascular implant 102, a delivery catheter 104 for delivering the intravascular implant 102 to a target site in the vasculature of the patient, and a pusher member assembly 106 (shown in **Figs. 7A** and **7D****)** slidably disposed in the delivery catheter 104 for coaxially carrying the intravascular implant 102.

The implant delivery system 100 may be used in an "over-the-wire" configuration, wherein the delivery catheter 104 is introduced into the patient over a guidewire (not shown) that has been previously introduced, and the delivery catheter 104 extends over the entire length of the guidewire (not shown). Alternatively, the implant delivery system 100 may be used in a "rapid-exchange" configuration, where a guidewire (not shown) extends through only a distal portion of the delivery catheter 104 from a guidewire port (not shown). In other alternative embodiments, the implant delivery system 100 may be introduced into the patient after a guidewire had been withdrawn, leaving a guide sheath for the delivery catheter 104 to navigate through the vasculature of the patient within the guide sheath.

As best illustrated in **Figs. 7A** and **7D****,** the intravascular implant 102 takes the form of, e.g., a stent, a stent graft, flow-diverter, vaso-occlusive device, vena cava filter, etc. Intravascular implant 102 generally comprises a tubular implant body 108 having a proximal portion 110 and a distal portion 112, and a central implant lumen 114 axially extending entirely through the tubular implant body 108. The tubular implant body 108 is composed of a resilient material, such that the intravascular implant 102 assumes a compressed delivery configuration when radially constrained within the delivery catheter 104, and automatically assumes an expanded deployed configuration when released from the constraints of the delivery catheter 104 (i.e., when the intravascular implant 102 is deployed from the delivery catheter 104. The tubular implant body 108 may be composed of a variety of biocompatible materials, such as, e.g., stainless steel, elgiloy, nickel, titanium, nitinol, shape memory polymers, or combinations thereof, and may be constructed using well-known techniques, such as by etching or cutting a pattern from a tube or sheet of stent material, or by weaving/braiding one or more wires or ribbons into a desired shape and pattern. The intravascular implant 102 may include further components that are welded, bonded or otherwise engaged to one another, and may optionally include a non-porous, non-permeable biocompatible material, cover or the like. The intravascular implant 102 may optionally comprise bioactive or therapeutic agents carried by, or coated on the inner surface or outer surface of the, tubular implant body 108.

The delivery catheter 104 may, e.g., comprise a length about 50cm-300cm, and typically about 60cm-200cm. The delivery catheter 104 is configured for accessing a body lumen, such as a blood vessel (e.g., an intracranial vein or a carotid artery), for a desired treatment in a target site. For example, the target site may be within a small diameter blood vessel having a 2-12mm lumen diameter and accessible by way of a tortuous vessel path, which may involve multiple vessel turns and multiple vessel branches. In such cases, the delivery catheter 12 has a small suitable diameter and flexible construction.

The delivery catheter 104 comprises an elongate sheath body 116 having a proximal portion 118 and a distal portion 120, and an inner sheath lumen 122 (best shown in **Fig. 7A****)** axially extending entirely through the sheath body 116. The inner sheath lumen 122 terminates in a distal port 124 at the end of the distal portion 120 of the sheath body 116. The proximal portion 118 of the sheath body 116 remains outside of the patient and accessible to the operator when the implant delivery system 100 is in use, while the distal portion 120 of the sheath body 116 is sized and dimensioned to reach remote locations of a vasculature to deliver the intravascular implant 102 to a target location in a patient's body, such as an occlusion in a blood vessel, in a blood vessel adjacent to an aneurysm neck, a bifurcated blood vessel, or the like. The inner sheath lumen 122 is sized to accommodate longitudinal movement of the radially contracted intravascular implant 102 and the pusher member assembly 106.

The delivery catheter 104 comprises a proximal adapter 126 affixed to the proximal portion 118 of the sheath body 116. The proximal adapter 126 comprises a central bore 128 (shown in phantom) in communication with the inner lumen 122 of the sheath body 116. The central bore 128 terminates in a proximal port 130 for allowing loading of the pusher member assembly 106 into the delivery catheter 104. The proximal adapter 126 further comprises at least one fluid port 132 in fluid communication with the inner lumen 122 for introducing fluids into the delivery catheter 104 in order to hydrate the pusher member assembly 106 and the intravascular implant 102. The delivery catheter 104 further comprises a tapered radiopaque marker 134 disposed on the distal portion 120 of the sheath body 116 proximate the distal port 124, such that the location of the distal tip of the delivery catheter 104 within the patient's vasculature system, or relative to the partially or fully deployed intravascular implant 102, can be identified using medical imaging technology (e.g., fluoroscopy). The delivery catheter 104 further comprises an atraumatic distal tip 136 affixed to the distal tip of the sheath body 116.

The delivery catheter 104 may include one or more, or a plurality of regions along its length having different configurations and/or characteristics. For example, the distal portion 120 of the sheath body 116 may have an outer diameter less than the outer diameter of the proximal portion 118 to reduce the profile of the distal portion 120 and facilitate navigation in tortuous vasculature. Furthermore, the distal portion 120 may be more flexible than the proximal portion 118. Generally, the proximal portion 118 may be formed from material that is stiffer than the distal portion 120, so that the proximal portion 118 has sufficient pushability to advance through the patient's vascular system, while the distal portion 120 may be formed of a more flexible material, so that the distal portion 120 may remain flexible and track more easily over a guidewire to access remote locations in tortuous regions of the vasculature. In some instances, the proximal portion 118 may include a reinforcement layer, such as a braided layer or coiled layer, to enhance the pushability of the delivery catheter 104. The delivery catheter 104 may include a transition region between the proximal portion 118 and the distal portion 120.

The sheath body 116 may be composed of suitable polymeric materials, metals and/or alloys, such as polyethylene, stainless steel or other suitable biocompatible materials or combinations thereof. Examples of suitable metals and metal alloys can include stainless steel, such as 304V, 304L, and 316L stainless steel; nickel-titanium alloy such as a superelastic (i.e., pseudoelastic) or linear elastic nitinol; nickel-chromium alloy; nickel-chromium-iron alloy; cobalt alloy; tungsten or tungsten alloys; tantalum or tantalum alloys, gold or gold alloys, MP35-N (having a composition of about 35% Ni, 35% Co, 20% Cr, 9.75% Mo, a maximum 1% Fe, a maximum 1% Ti, a maximum 0.25% C, a maximum 0.15% Mn, and a maximum 0.15% Si); or the like; or other suitable metals, or combinations or alloys thereof. Examples of some suitable polymers can include, but are not limited to, polyoxymethylene (POM), polybutylene terephthalate (PBT), polyether block ester, polyether block amide (PEBA), fluorinated ethylene propylene (FEP), polyethylene (PE), polypropylene (PP), polyvinylchloride (PVC), polyurethane, polytetrafluoroethylene (PTFE), polyether-ether ketone (PEEK), polyimide, polyamide, polyphenylene sulfide (PPS), polyphenylene oxide (PPO), polysufone, nylon, perfluoro(propyl vinyl ether) (PFA), polyether-ester, polymer/metal composites, or mixtures, blends or combinations thereof.

The sheath body 116 may include a braided-shaft construction of stainless steel flat wire that is encapsulated or surrounded by a polymer coating. By way of nonlimiting example, HYDROLENE is a polymer coating that may be used to cover the exterior portion of the sheath body 116. Of course, the implant delivery system 100 is not limited to a particular construction or type of delivery catheter 104 and other constructions known to those skilled in the art may be used for the sheath body 116 of the delivery catheter 12. The inner sheath lumen 122 may be advantageously coated with a lubricious coating (not shown), such as PTFE, to reduce frictional forces between the sheath body 116 and the pusher member assembly 106 and intravascular implant 102 when axially translated within the inner lumen 122.

The pusher member assembly 114 is slidably disposed in the inner lumen 122 of the delivery catheter 104, with the intravascular implant 102 coaxially disposed between the delivery catheter 104 and the pusher member assembly 114. The pusher member assembly 114 is configured for engaging the intravascular implant 102 when the pusher member assembly 114 is axially translated relative to the delivery catheter 104 for delivery of the intravascular implant 102 into a target site of a patient or resheathing the intravascular implant 102 back into the delivery catheter 104. The interface between the pusher member assembly 114 and the intravascular implant 102 will be described in further detail below.

The pusher member assembly 114 generally comprises an elongate pusher member in the form of a delivery wire 138 having a proximal portion 140 that extends proximally from the proximal adapter 126 of the delivery catheter 104 and which a physician can grasp to axially translate the pusher member assembly 114 within the inner lumen 122 of the delivery catheter 104, a distal portion 142 that carries the intravascular implant 102, and a transition portion 144 between the proximal portion 140 and the distal portion 142 for facilitating tracking of the pusher member assembly 114 within the inner lumen 122 of the delivery catheter 104.

The delivery wire 138 may be composed of a conventional guidewire, torqueable cable tube, or a hypotube. In either case, there are numerous materials that can be used for the delivery wire 138 to achieve the desired properties that are commonly associated with medical devices. Some examples can include metals, metal alloys, polymers, metal-polymer composites, and the like, or any other suitable material (e.g., nickel-titanium alloy, stainless steel, a composite of nickel-titanium alloy and stainless steel). In some cases, the delivery wire 138 can be made of the same material along its length, or in some embodiments, can include portions or sections made of different materials. In some embodiments, the material used to construct the delivery wire 138 is selected to impart varying flexibility and stiffness characteristics to different portions of the delivery wire 138. For example, the proximal portion 140, distal portion 142, and transition portion 144 of the delivery wire 138 may be formed of different materials, for example materials having different moduli of elasticity, resulting in a difference in flexibility. For example, the proximal portion 140 can be formed of stainless steel, and the distal portion 142 and intermediate portion 144 can be formed of a nickel-titanium alloy. However, any suitable material or combination of material may be used for the delivery wire 138, as desired.

In the illustrated embodiment, the pusher member assembly 106 further comprises a coil 146 affixed to the transition portion 144 of the delivery wire 138. The diameter of the transition portion 144 with the coil 146 is closely toleranced to the diameter of the inner sheath lumen 122 of the delivery catheter 104 to facilitate stable tracking of the pusher member assembly 106 through the inner sheath lumen 122, while the coil 146 provides columnar support to the pusher member assembly 106 just proximal to the distal portion 142 on which the intravascular implant 102 is disposed while maintaining lateral flexibility of the pusher member assembly 106. The diameter of the distal portion 142 is decreased, thereby providing a platform on which the intravascular implant 102, in its compressed delivery configuration, as well as other components of the pusher member assembly 106 (described in further detail below), are disposed, such that the outer diameter of the compressed intravascular implant 102 and the distal portion of the pusher member assembly 106 are also closely toleranced to the diameter of the inner lumen 122 of the delivery catheter 104 **[is this correct?].**

The pusher member assembly 106 further comprises an atraumatic distal tip member 148 affixed to the distal end of the delivery wire 138. The distal tip member 148 allows for vessel selection and facilitates navigation of the implant delivery system 100 to the targeted implantation site. The distal tip member 148 may be pre-shaped to assume a curved geometry (and in the preferred embodiment, J-shaped geometry) when unconstrained, and to assume a straight geometry when constrained. Alternatively, the distal tip member 148 may be floppy, e.g., composed of a soft coil member. The distal tip member 148 may optionally be radiopaque to aid in its visualization using medical imaging technology (e.g., fluoroscopy). The pusher member assembly 106 further comprises a protective device 150 affixed to the delivery wire 138 via one or more locking members 152 for covering the distal portion of the intravascular implant 102.

The pusher member assembly 106 further comprises a proximal bumper 154 affixed to the distal portion 142 just distal to the transition portion 144 of the delivery wire 138, and a distal bumper 156 affixed to the distal portion 142 of the delivery wire 138 distal to the proximal bumper 154 (e.g., via epoxy bonding), thereby creating an annular space 158 between the bumpers 154, 156. The bumpers 154, 156 may be composed of a suitable biocompatible material, such as stainless steel or nitinol. Each of the bumpers 154, 156 is disk-shaped, and is sized to slide within the inner sheath lumen 122 of the delivery catheter 104, although in alternative embodiments, the bumpers 154, 156 may have any suitable shape. In the illustrated embodiment, the diameter of the proximal bumper 154 is closely toleranced with the diameter of the inner sheath lumen 122, while the distal bumper 156 has a tip that is tapered towards the distal direction to minimize tissue trauma. The outer surfaces of the bumpers 154, 156 may impart low-friction due to the material from which they are formed. Alternatively, or additionally, the outer surfaces of the bumpers 154, 156 may be coated with a lubricious coating, e.g., polytetrafluoroethylene (PTFE), hereby facilitating movement of the bumpers 154, 156 through the inner sheath lumen 122.

As best illustrated in **Fig. 7C****,** the pusher member assembly 106 further comprises a friction pad 162 coaxially disposed around the delivery wire 138 within the annular space 158 between the bumpers 154, 156. In particular, the friction pad 162 comprises a tubular pad body 164 having a proximal portion 166 and a distal portion 168, and a central pad lumen 170 axially extending entirely through the tubular pad body 164 and in which the delivery wire 138 is disposed. The distal surface of the proximal bumper 154 serves to abut the proximal portion 166 of the friction pad 162, such that the friction pad 162 axially translates with the delivery wire 138 as the pusher member assembly 106 is translated relative to the delivery sheath 104 in the distal direction (e.g., during deployment of the intravascular implant 102 from the delivery catheter 104). In contrast, the proximal surface of the distal bumper 154 serves to abut the distal portion 168 of the friction pad 162, such that the friction pad 162 axially translates with the delivery wire 138 as the pusher member assembly 106 is translated relative to the delivery sheath 104 in the proximal direction (e.g., during resheathing of the intravascular implant 102 within the delivery catheter 104).

One or both of the bumpers 154, 156 may be radiopaque, and thus, also serve as markers that may assist in locating the intravascular implant 102 relative to the delivery catheter 104 using medical imaging technology (e.g., fluoroscopy). For example, such radiopaque proximal bumper 154 may indicate the proximal end of the intravascular implant 102, so that full deployment of the intravascular implant 102 from the delivery catheter 104 can be confirmed, while such radiopaque distal bumper 156 may indicate the point-of-no-return as the intravascular implant 102 is being deployed from the delivery catheter 104, after which the intravascular implant 102 may no longer be resheathed into the delivery catheter 104. To this end, the bumpers 154, 156 may be composed of a suitably radiopaque material, such as, e.g., platinum, gold, tungsten, or alloys thereof or other metals. The pusher member assembly 106 may optionally comprise additional radiopaque elements anywhere along its length to facilitate its visualization using medical imaging technology (e.g., fluoroscopy).

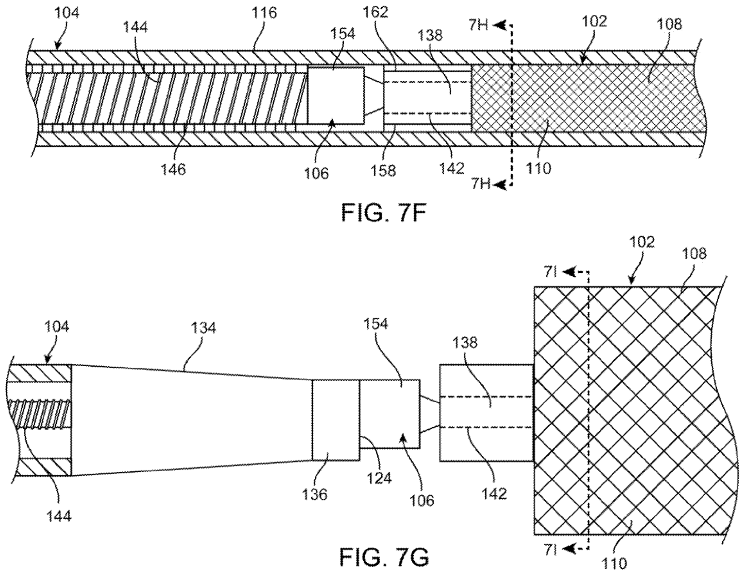

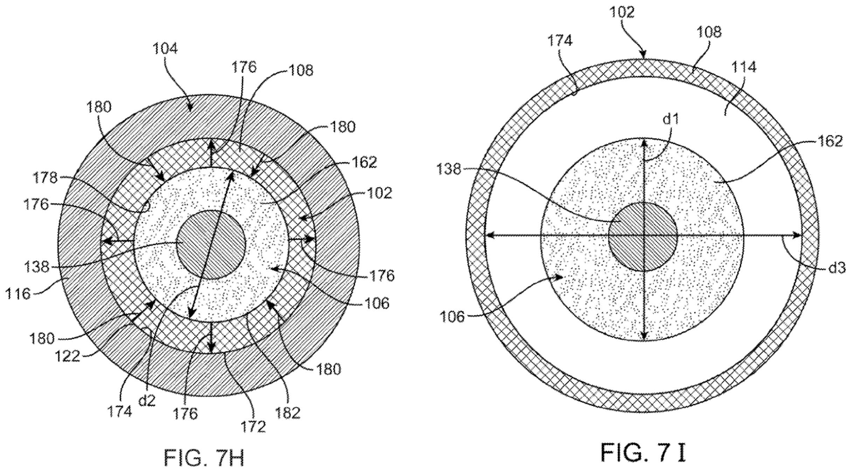

As illustrated in **Figs. 7F** and **7H****,** the intravascular implant 102, in its compressed delivery configuration, is disposed within the inner lumen 122 of the delivery catheter 104 in a coaxial arrangement with the distal portion of the pusher member assembly 106. In the illustrated embodiment, the intravascular implant 102 extends from the proximal end of the friction pad 162 to the distal tip of the delivery wire 138, such that the proximal portion 110 of the tubular implant body 108 of the intravascular implant 102 and the friction pad 162 are in a coaxial arrangement; that is, the proximal portion 110 of tubular implant body 108 is disposed between the outer surface 172 of the friction pad 162 and the inner surface 174 of the delivery catheter 104. In the illustrated embodiment, the intravascular implant 102 extends from only a portion of the friction pad 162 to the distal tip of the delivery wire 138, although it should be appreciated that the friction pad 162 may extend along the entirety of the friction pad 162 to the distal tip of the delivery wire 138 or to a point proximal to the distal tip of the delivery wire 138, although the intravascular implant 102 may alternatively extend over the entirety of the friction pad 162. In contrast, as illustrated in **Figs. 7G** and **7I****,** the intravascular implant 102, in its expanded deployed configuration, is no longer disposed within the inner lumen 122 of the delivery catheter 104, but may still be in a coaxial arrangement with the distal portion of the pusher member assembly 106.

The friction pad 162 is configured for cooperating with the intravascular implant 102, such that the pusher member assembly 106 engages the intravascular implant 102 when the intravascular implant 102 is in the compressed delivery configuration within the inner lumen 122 of the delivery catheter 104 (shown in **Figs. 7F** and **7G****),** and releases the intravascular implant 102 when the intravascular implant 102 is in the expanded deployed configuration outside of the inner lumen 122 of the delivery catheter 104 (shown in **Figs. 7G** and **7I****).** To the end, the friction pad 162 is elastically deformable, such that it can alternately transition between a radially compressed state (shown in **Figs. 7F** and **7G****)** and a radially expanded state (shown in **Fig. 7H****).** The cross-sectional dimension of the friction pad 162, when in the radially expanded state (e.g., diameter d1 illustrated in **Fig. 7I****),** is greater than the cross-sectional dimension of the inner lumen 114 of the intravascular implant 102 (e.g., diameter d2 illustrated in **Fig. 7H****)** when in the compressed delivery configuration within the inner lumen 122 of the delivery catheter 104, thereby ensuring that the friction pad 162, when in the radially compressed state within the inner lumen 114 of the compressed intravascular implant 102, frictionally engages the intravascular implant 102. In contrast, the cross-sectional dimension of friction pad 162, when in the radially expanded state (e.g., diameter d1 illustrated in **Fig. 7I****),** is less than the cross-sectional dimension of the inner lumen 114 of the intravascular implant 102 when in the expanded deployed configuration (e.g., diameter d3 illustrated in **Fig. 7I****)** outside of the inner lumen 122 of the delivery catheter 104, thereby ensuring that the friction pad 162, when in the radially expanded state within the inner lumen 114 of the expanded intravascular implant 102, releases the intravascular implant 102.

In particular, as best illustrated in **Fig. 7H****,** when the intravascular implant 102 is in the compressed delivery configuration within the inner sheath lumen 122, the friction pad 162 is disposed within the central implant lumen 114 of the intravascular implant 102 (i.e., coaxially disposed between the delivery wire 138 and the intravascular implant 102) in the radially compressed state, such that friction pad 162 imparts a radially outward force 176 against an inner surface 178 of the intravascular implant 102, while the inner sheath lumen 122 imparts a radially inward force 180 against an outer surface 182 of the intravascular implant 102. In this manner, the proximal portion 162 of the intravascular implant 102 is frictionally engaged (i.e., gripped) by the pusher member assembly 106, such that the entire intravascular implant 102 moves in unison with the pusher member assembly 106 as the pusher member assembly 106 and delivery catheter 104 are axially translated relative to each other (e.g., by translating the delivery catheter 104 in a proximal direction while holding the pusher member assembly 106 in place) to deploy the intravascular implant 102 out of the distal port 124 of the delivery catheter 104 and into its expanded deployed configuration within the blood vessel.

In contrast, as best illustrated in **Fig. 7I****,** when the intravascular implant 102 is in the expanded deployed configuration outside of the inner lumen 122 of the delivery catheter 104, the friction pad 162, although coaxially disposed between the delivery wire 138 and the intravascular implant 102 in the radially expanded state, does not impart a radially outward force against the inner surface 178 of the intravascular implant 102. In this manner, the intravascular implant 102 is no longer frictionally engaged (i.e., gripped) by the pusher member assembly 106, such that the pusher assembly 106 moves independently of the deployed intravascular implant 102 as the pusher member assembly 106 and delivery catheter 104 are axially translated relative to each other (e.g., by translating the pusher member assembly 106 in a proximal direction while holding the delivery catheter 104 in place) to leave the deployed intravascular implant 102 in place as the pusher member assembly 106 is fully retracted within the distal port 124 of the delivery catheter 104.

Referring back to **Figs. 7A-7D****,** the friction pad 162 not only facilitates deployment of the intravascular implant 102, it serves to facilitate resheathing of the stent 102 within the inner lumen 122 of the delivery catheter 104 (e.g., by translating the delivery catheter 104 in the distal direction while holding the pusher member assembly 106 in place). As long as the intravascular implant 102 has not been deployed from the delivery catheter 104 past the point-of-no-return (i.e., as long as distal bumper 156 remains in the inner lumen 122 of the delivery catheter 104, indicating that the friction pad 162, which will tend to expand outside of the constraints of the delivery catheter 104, is fully within the inner lumen 122 of the delivery catheter 104), the intravascular implant 102, facilitated by the friction pad 162, may be resheathed back into its compressed delivery configuration within the delivery catheter 104.

In the illustrated embodiment, although the friction pad 162 is generally cylindrical in nature, in alternative embodiments, the friction pad 162 may have non-circular cross-sectional profiles (e.g., octagonal). In the illustrated embodiment, the friction pad 162 is composed of an elastomeric base material. Such elastomeric base material, may be a high-friction polymer, such as, e.g., polyether block amide (PEBA), to facilitate frictional engagement with the intravascular implant 102. Alternatively, other types of suitable polymers for the elastomeric base material can include, but are not limited to, polyoxymethylene (POM), polybutylene terephthalate (PBT), polyether block ester, fluorinated ethylene propylene (FEP), polyethylene (PE), polypropylene (PP), polyvinylchloride (PVC), polyurethane, polytetrafluoroethylene (PTFE), polyether-ether ketone (PEEK), polyimide, polyamide, polyphenylene sulfide (PPS), polyphenylene oxide (PPO), polysufone, nylon, perfluoro(propyl vinyl ether) (PFA), polyether-ester, polymer/metal composites, or mixtures, blends or combinations thereof.

In one alternative embodiment, the friction pad 162 may be coated with a high friction, tacky material. In another alternative embodiment, the friction pad 162, the outer surface of the friction pad 162 may be modified to provide the desired resistance for frictionally engaging the intravascular implant 102. For example, at least a portion of the outer surface of the friction pad 162 may be roughened by any suitable technique, such as grit blasting, plasma treatment, or knurling, or lateral grooves, protuberances, or rings may be formed on at least a portion of the outer surface of the friction pad 162.

In the illustrated embodiment, although a single friction pad 162 is disposed within the annular space 158 between the bumpers 154, 156, in alternative embodiments, multiple friction pads may be disposed within the annular space 158 between the bumpers 154, 156. Furthermore, although the friction pad 162 is designed to uniformly frictionally engage the length of the intravascular implant 102 between the bumpers 154, 156, the friction pad 162 or friction pads may non-uniformly frictionally engage only portions of the intravascular implant 102 between the bumpers 154, 156.

As previously discussed, it is desirable that the tubular pad body 164 have a relatively low radial strength, such that the friction pad 162 is radially compressible/expandable enough to impart sufficient amount of radially outward force to frictionally engage the intravascular implant 102, have a relatively low bending stiffness, such that the friction pad 162 will also more easily track through bends in the vasculature of the patient, and have a relatively high columnar strength to prevent or at least minimize radial outward bulging of the tubular pad body 164 in the presence of increased resistance to the translation of the intravascular implant 102 within the inner lumen 122 of the delivery catheter 104 during deployment and/or resheathing of the intravascular implant 102.

It should also be appreciated that the columnar strength of the tubular pad body 164 may be characterized in terms of axial strain (i.e., the change in length of the tubular pad body 164 in the presence of a unit axial force (or axial stress) divided by the length of the tubular pad body 164 in the absence of an axial force (or axial stress), whereas the radial strength of the tubular pad body 164 may be characterized in terms of radial strain (i.e., the change in diameter of the tubular pad body 164 in the presence of a unit radial force (or radial stress) divided by the diameter of the tubular pad body 164 in the absence of a radial force (or radial stress).

Significantly, the friction pad 162 is constructed in a manner that it has sufficiently high columnar strength and sufficiently low radial strength and bending stiffness, such that radial outward bulging of the friction pad 162 is minimized in the presence of an increased resistance to the translation of the intravascular implant 102 relative to the lumen 122 of the delivery catheter 104 during deployment and/or resheathing of the intravascular implant 102, while allowing the friction pad 162 to frictionally engage the intravascular implant 102 and easily track through the vasculature of the patient.

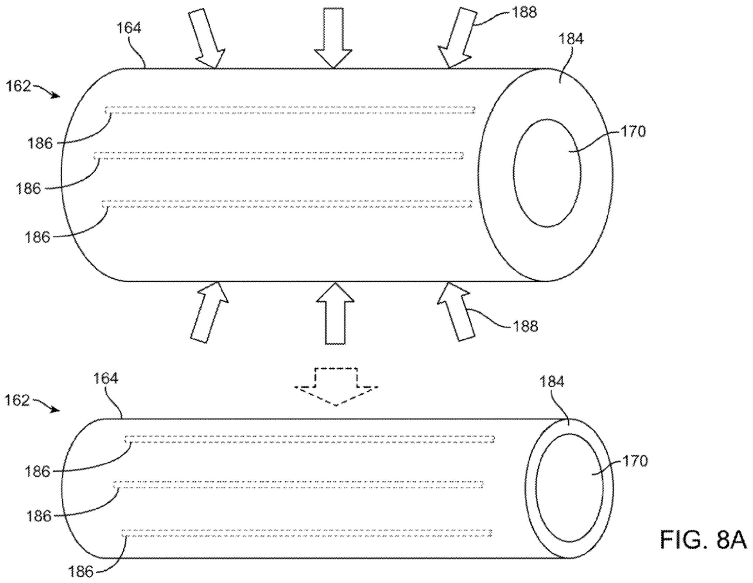

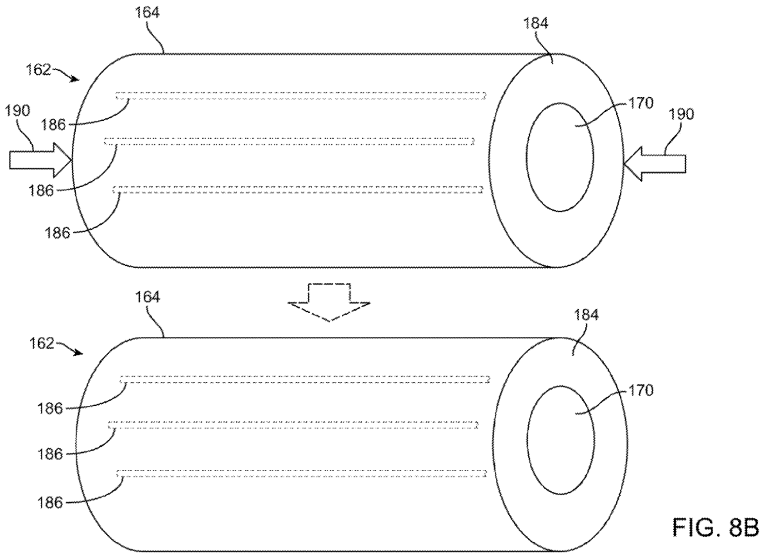

In particular, as illustrated in **Figs. 8A** and **8B****,** the tubular pad body 164 of the friction pad 162 comprises an elastomeric base material 184 that forms the friction pad 162 and at least modulating element 186 (a plurality of modulating elements 186 shown) axially extending within the elastomeric base material 184. The modulating elements 186 may be embedded within the elastomeric base material 184 using any suitable process, such as, e.g., through an extrusion process, through overmolding, or through a lamination process. In the illustrated embodiment, the modulating elements 186 are elongated and extend in a direction parallel to the longitudinal axis of the tubular pad body 164. However, it should be appreciated that the modulating elements 186 may extend obliquely relative to the longitudinal axis of the tubular pad body 164 (e.g., not greater than fifteen degrees from the longitudinal axis of the tubular pad body 164. As discussed in further detail below, the modulating elements 186 serve to modulate the mechanical properties of the tubular pad body 164, and thus, the mechanical properties of the material from which the modulating elements 186 are composed are substantially differ from the mechanical properties of the material from the which the elastomeric base material 184 is composed.

Significantly, the modulating elements 186 are configured for either increasing the columnar strength of the tubular pad body 164, without prohibitively increasing the radial strength of the tubular pad body 164 (i.e., the radial strength of the tubular pad body 164 will not be so great that the friction pad 162 will not be resilient enough to have enough radially outward force to frictionally engage the intravascular implant 102) and increasing the bending stiffness of the tubular pad body 164, or decreasing the radial strength or lateral bending stiffness of the tubular pad body 164 without prohibitively decreasing the columnar strength of the tubular pad body 164 (i.e., the columnar strength of the tubular pad body 164 will not be so weak that the friction pad 162 will radially outwardly bulge in the presence of increased resistance to the translation of the intravascular implant 102 within the inner lumen 122 of the delivery catheter 104 during deployment and/or resheathing of the intravascular implant 102).

In one embodiment, the modulating elements 186 increase the ratio of the columnar strength over the radial strength of the tubular pad body 164 (i.e., the ratio of the columnar strength over the radial strength of the tubular pad body 164 with the axially extending elements 186 is substantially greater than the ratio of the columnar strength over the radial strength of the tubular pad body 164 if the axially extending element(s) were replaced with the same elastomeric material 184 that would otherwise occupy the space in the tubular pad body 164 where the axially extending elements 186 otherwise be) is increased. It should be appreciated that, because the lateral bending stiffness of the tubular pad body 164 is directly correlated to the radial strength of the tubular pad body 164, as the ratio of the columnar strength over the radial strength of the tubular pad body 164 increases, the ratio of the columnar strength over the lateral bending stiffness of the tubular pad body 164 will likewise increase in much the same manner that a solid cylinder composed of a rigid material would significantly increase the lateral bending stiffness of the solid cylinder.

Preferably, the number, cross-sectional size, cross-sectional shape, orientation, and composition of the modulating elements 186 are selected to maximize the volume of the elastomeric base material 184. That is, the total volume of the modulating elements 186 necessary to achieve the desired ratio of the columnar strength over the radial strength of the tubular pad body 164 should be minimized, so as to minimize any deleterious effect on the radial strength (and bending stiffness) of the tubular pad body 164 in the case where the modulating elements 186 serve to increase the columnar strength of the tubular pad body 164, or so as to minimize any deleterious effect on the columnar strength of the tubular pad body 164 in the case where the modulating elements 186 serve to decrease the radial strength (and bending stiffness) of the tubular pad body 164. In other words, the modulating elements 186 are designed to tune the mechanical properties (i.e., the columnar strength and radial strength) of the tubular pad body 164 as opposed to dominating the mechanical properties of the tubular pad body 164. Thus, it is preferred that the number and cross-sectional size of the modulating elements 186 be minimized as much as possible. It is also preferred that the thickness of the cross-sectional shapes of the modulating elements 186 be as thin as possible. It should be appreciated that as the difference in mechanical properties of the material from which the modulating elements 186 are composed and the mechanical properties of the material from the which the elastomeric base material 184 is composed increases, the modulation of the tubular pad body 164 by the modulating elements 186 will increase, and thus, the cross-sectional size of the modulating elements 186 may be decreased.

As illustrated in **Fig. 8A****,** due to the modulation of the tubular pad body 164 by the modulating elements 186, the diameter of the friction pad 162 will be reduced by a desired amount (e.g., from diameter d1 to diameter d2) in response to a radially compressive force 188 (i.e., corresponding to the radially inward force 180 imparted by the inner sheath lumen 122 of the delivery catheter 104 against the outer surface 182 of the intravascular implant 102 illustrated in **Fig. 7H****).** In contrast, as illustrated in **Fig. 8B****,** the length or diameter of the friction pad 162 (e.g., diameter d2) will not substantially change in response to an axially compressive force 190 (i.e., the force caused by the increased resistance to the translation of the intravascular implant 102 relative to the lumen 122 of the delivery catheter 104 during deployment and/or resheathing of the intravascular implant 102).

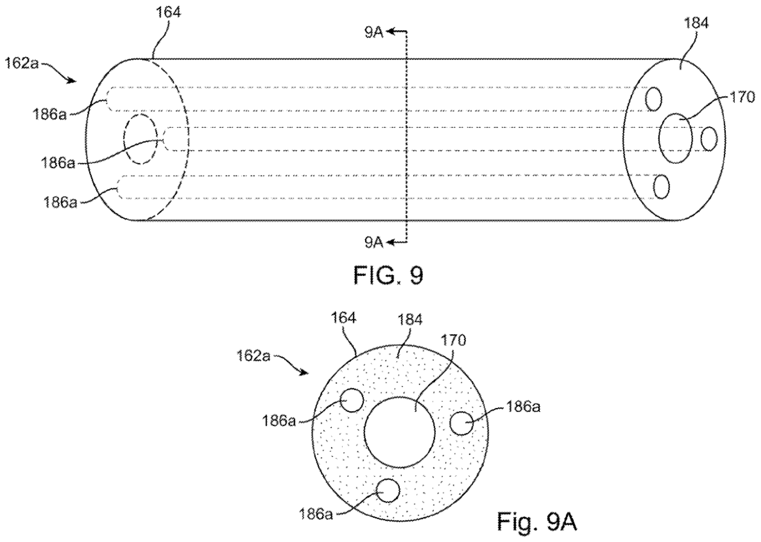

Referring to **Fig. 9****,** the tubular pad body 164a of one embodiment of a friction pad 162a comprises three elongated modulating elements 186a that axially extend entirely through the elastomeric base material 184. The modulating elements 186a are circumferentially arranged within the elastomeric base material 184, and in particular, are circumferentially spaced 120 degrees from each other. Although the tubular pad body 164a comprises three modulating elements 186a, it should be appreciated that the tubular pad body 164a may comprise any number of modulating elements 186a. However, it is preferred that the modulating elements 186a be equally circumferentially spaced from each other. For example, if the tubular pad body 164a alternatively comprises four modulating elements 186a, it is preferred that they be circumferentially spaced 90 degrees from each other. In the illustrated embodiment, each of the modulating elements 186a is cylindrical (i.e., it has a circular cross-section), as illustrated in **Fig. 9A****.**

In alternative embodiments, one or more of the modulating elements 186a may have other cross-sections, such as triangular, rectangular, oval, ellipsoidal, etc. Furthermore, although in the illustrated embodiment, the cross-sectional size of each of the modulating elements 186a is uniform along the length of the respective modulating element, the cross-sectional size of one or more of the modulating elements 186 may vary along the length of the respective modulating element. Furthermore, although the cross-sectional sizes of all of the modulating elements 186a are illustrates as being the same, the cross-sectional sizes of the modulating elements 186a may different from each other.

It should be appreciated that the cross-sectional sizes and composition of the modulating elements 186a may be selected to provide a desired ratio of the columnar strength over the radial strength (or bending stiffness) of the tubular pad body 164a. That is, the larger the cross-sectional size and/or higher the durometer (or modulus of elasticity) of the modulating elements 186a, the greater the ratio of the columnar strength over the radial strength of the tubular pad body 164a, while the lesser the cross-sectional size and/or lower the durometer (or modulus of elasticity) of the modulating elements 186a, the lesser the ratio of the columnar strength over the radial strength of the tubular pad body 164a.

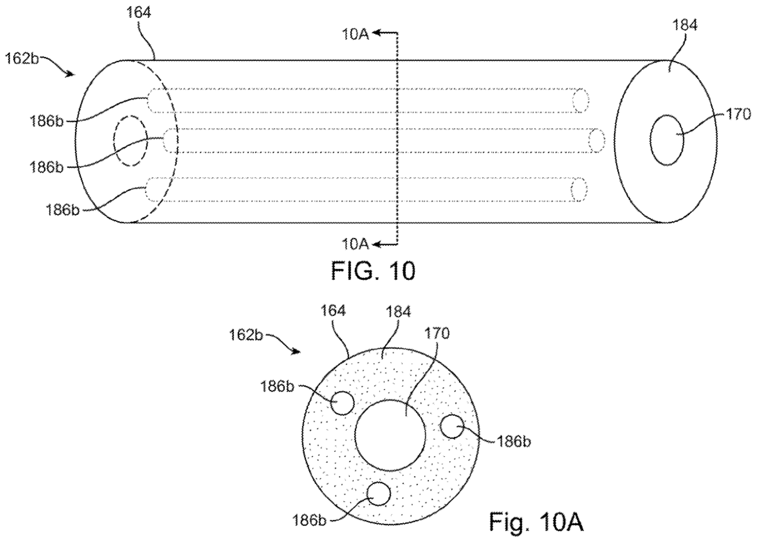

Referring to **Fig. 10****,** the tubular pad body 164b of another embodiment of a friction pad 162b differs from the tubular pad body 164a of the friction pad 162a of **Fig. 9** in that, instead of comprising elongated modulating elements 186a that extend entirely through the elastomeric base material 184, the tubular pad body 164b comprises three elongated modulating elements 186b that axially extend only partially through the elastomeric base material 184. Thus, the modulating elements 186b do not axially extend all the way to a proximal facing surface 192 of the tubular pad body 164b or to the distal facing surface 194 of the tubular pad body 164b. As with the modulating elements 186a of the tubular pad body 164a illustrated in **Fig. 9A****,** each of the modulating elements 186b of the tubular pad body 164b is cylindrical (i.e., it has a circular cross-section), as illustrated in **Fig. 10A****.**

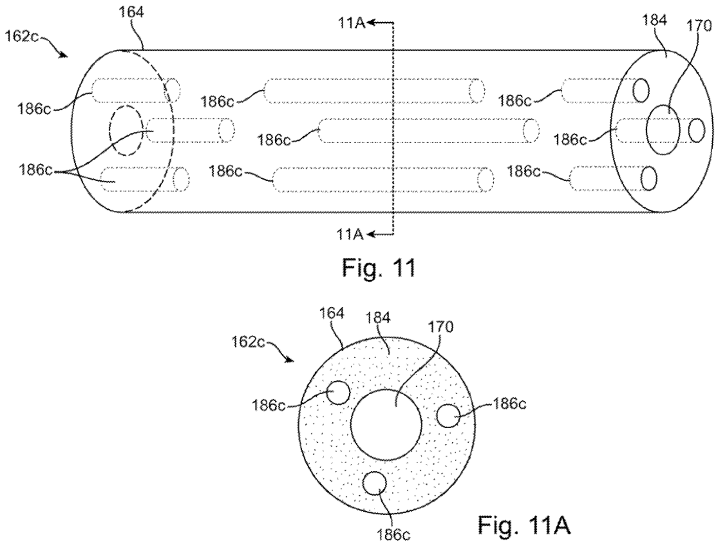

Referring to **Fig. 11****,** the tubular pad body 164c of still another embodiment of a friction pad 162c differs from the tubular pad body 164a of the friction pad 162a of **Fig. 9** in that, instead of comprising a single elongated modulating element 186a that axially extends within the elastomeric base material 184 at each circumferential position, the tubular pad body 164c comprises a series of elongated modulating elements 186c that axially extend within the elastomeric material 184 at each circumferential position. In the illustrated embodiment, three modulating elements 186c (a relatively long center modulating element 186c flanked by two relatively short modulating elements 186c) axially extend within the elastomeric base material 184 at each circumferential position. As with the modulating elements 186a of the tubular pad body 164a illustrated in **Fig. 9A****,** each of the modulating elements 186c of the tubular pad body 164c is cylindrical (i.e., it has a circular cross-section), as illustrated in **Fig. 11A****.**

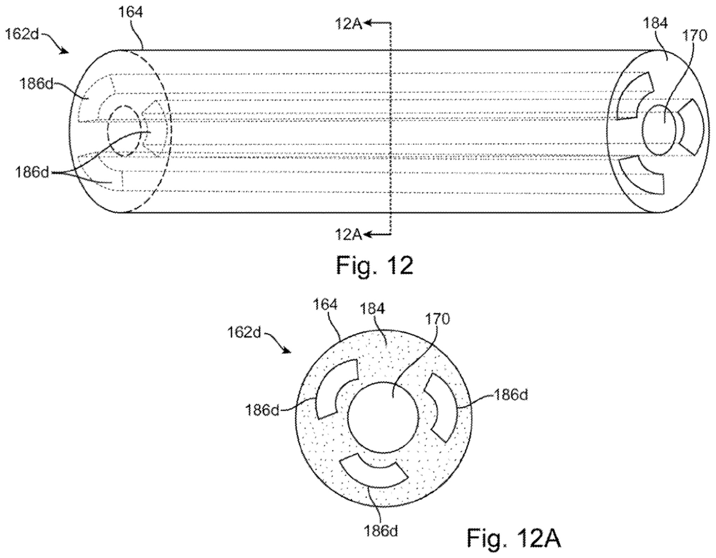

Referring to **Fig. 12****,** the tubular pad body 164d of still another embodiment of a friction pad 162d differs from the tubular pad body 164a of the friction pad 162a of **Fig. 9** in that, instead of comprising elongated modulating elements 186a having circular cross-sections, the tubular pad body 164d comprises elongated modulating elements 186d having arcuate cross-sections, as best illustrated in **Fig. 12A****.** In this manner, the acuate cross-sectional shape of the elongated modulating elements 186d better conforms to the circumference of the elastomeric base material 184. In this manner, the volume of the elongated modulating elements 186d may be minimized, thereby maximizing the volume of the elastomeric base material 184.

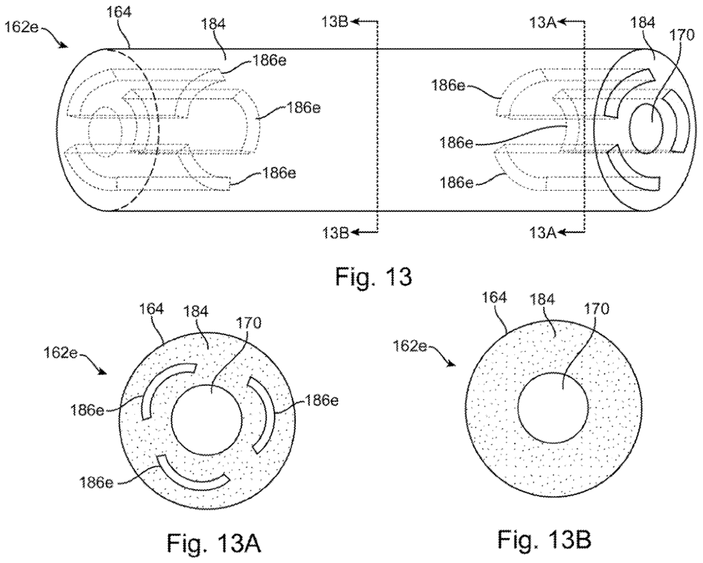

Referring to **Fig. 13****,** the tubular pad body 164e of still another embodiment of a friction pad 162e differs from the tubular pad body 164d of the friction pad 162a of **Fig. 9** in that the tubular pad body 164e comprises modulating elements 186e that are not elongated. Furthermore, the thickness of the arcuate cross-sections of the modulating elements 186 are thinner commensurate with the reduced length of the modulating elements 186e, as best illustrated in **Fig. 13A****.** Furthermore, the modulating elements 186e are disposed only in the ends of the elastomeric base material 184, leaving the center of the elastomeric base material 184 free of any modulating elements 186e. As such, only the ends of the tubular pad body 164e are modulated by the modulating elements 186e, such that the center of the tubular pad body 164e remains unmodulated as illustrated in **Fig. 13B****,** while the ends of the tubular pad body 164e are modulated.

The composition of the modulating elements 186 of the tubular pad bodies 164 illustrated in **Figs. 9-13** will depend on the composition of the elastomeric base material 184. For example, the elastomeric base material 184 may have a relatively low durometer (e.g., in the range of 25A-35D) and thus, a relatively low modulus of elasticity (Young's modulus). Materials for such elastomeric base material 184 may include, e.g., Piothane^{®}, Isothane^{®}, Cronoflex^{®}, Cronoprene^{®}, silicone, Pebax^{®}, Versaflex^{®}, Picoflex^{®}.

In this case, such elastomeric base material 184 may provide sufficiently low enough radial strength (and thus high enough radial expandability/compressibility) to frictionally engage the intravascular implant 102 and low enough bending stiffness to allow the friction pad 162 to track through the vasculature of the patient. However, such elastomeric base material 184, by itself, may not provide sufficiently high enough columnar strength to prevent or otherwise minimize outward bulging of the tubular pad body 164 in response to the translation of the intravascular implant 102 within the inner lumen 122 of the delivery catheter 104 during deployment and/or resheathing of the intravascular implant 102.

In this case, it is preferred that the modulating elements 186 have a modulus of elasticity greater than the modulus of elasticity of the elastomeric base material 184, resulting in an increased ratio of the columnar strength over the radial strength of the tubular pad body 164 (by increasing the numerator (columnar strength) of the ratio). For example, each of the modulating elements 186 may be a solid beam composed of a suitable material, e.g., a metal or a metal alloy, such as stainless steel, nitinol, cobalt-chromium alloy, etc., or a high durometer polymer. In this manner, a sufficiently high enough columnar strength is provided to the tubular pad body 164 to prevent or otherwise minimize outward bulging of the tubular pad body 164 in response to the translation of the intravascular implant 102 within the inner lumen 122 of the delivery catheter 104 during deployment and/or resheathing of the intravascular implant 102, while not increasing the radial strength of the tubular pad body 164 to the extent that it cannot frictionally engage the intravascular implant 102 and not increasing the bending stiffness of the tubular pad body 164 to the extent that the friction pad 162 cannot easily track through the vasculature of the patient. Preferably, the modulating elements 186 are not rigidly coupled together (i.e., the only material that connects the modulating elements 186 together is the elastomeric base material 184), such that the modulating elements 186 essentially "laterally float" relative to each other. As a result, any deleterious effect on the radial strength and bending stiffness of the tubular pad body 164 is minimized.

As another example, the elastomeric base material 184 may have a relatively high durometer (e.g., greater than 35D), and thus, a relatively high modulus of elasticity (Young's modulus). Materials for such elastomeric base material 184 may include, e.g., Pebax^{®}, polyamide, polyurethane, polycarbonate, nylon, etc.

In this case, such elastomeric base material 184 may provide sufficient columnar strength to prevent or otherwise minimize outward bulging of the tubular pad body 164 in response to the translation of the intravascular implant 102 within the inner lumen 122 of the delivery catheter 104 during deployment and/or resheathing of the intravascular implant 102. However, such elastomeric base material 184, by itself, may not provide sufficiently low enough radial strength (and thus high enough radial expandability/compressibility) to frictionally engage the intravascular implant 102 or low enough bending stiffness to allow the friction pad 162 to easily track through the vasculature of the patient.

In this case, it is preferred that the modulating elements 186 have a modulus of elasticity (i.e., density) less than the modulus of elasticity of the elastomeric base material 184, resulting in an increased ratio of the columnar strength over the radial strength of the tubular pad body 164 (by decreasing the denominator (radial strength) of the ratio). For example, each of the modulating elements 186 may comprise a gel or air. In this manner, a sufficiently low enough radial strength is provided to the tubular pad body 164 to frictionally engage the intravascular implant 102, while not decreasing the columnar strength of the tubular pad body 164 to the extent that it outward bulging of the tubular pad 164 is not prevented or otherwise minimized during deployment and/or resheathing of the intravascular implant 102.

Although particular embodiments of the present inventions have been shown and described, it will be understood that it is not intended to limit the present inventions to the preferred embodiments, and it will be obvious to those skilled in the art that various changes and modifications may be made without departing from the spirit and scope of the present inventions. Thus, the present inventions are intended to cover alternatives, modifications, and equivalents, which may be included within the spirit and scope of the present inventions as defined by the claims.
The following clauses set out aspects of the present disclosure:
1. An intravascular implant delivery system, comprising: an intravascular implant having a compressed delivery configuration and an expanded deployed configuration, the intravascular implant comprising a tubular implant body and a central implant lumen axially extending through the tubular implant body; a delivery catheter having an elongate sheath body and an inner sheath lumen axially extending through the sheath body, the intravascular implant being disposed within the inner sheath lumen when in the compressed delivery configuration; and a pusher member assembly slidably disposed in the inner sheath lumen, the pusher member assembly comprising an elongate pusher member and a friction pad having a radially compressed state and a radially expanded state, the friction pad being disposed within the central implant lumen when in the radially compressed state, the friction pad comprising a tubular pad body and a central pad lumen axially extending through the tubular pad body, the pusher member disposed within the central pad lumen, the tubular pad body having an elastomeric base material that forms the tubular pad body and at least one modulating element embedded within and axially extending along the elastomeric base material, each of the at least one modulating element has different mechanical properties than mechanical properties of the elastomeric base material.
2. The intravascular implant delivery system of clause 1, wherein the pusher member assembly and delivery catheter can be axially translated relative to each other, and the friction pad is configured for frictionally engaging the intravascular implant as the pusher member assembly and delivery catheter are axially translated relative to each other, such that the intravascular implant and the pusher member assembly axially translate with each other in unison to deploy the intravascular implant from the inner sheath lumen into its expanded deployed configuration.
3. The intravascular implant delivery system of clause 2, wherein the friction pad is configured for frictionally engaging the intravascular implant as the pusher member assembly and the delivery catheter are axially translated relative to each other, such that the intravascular implant and the pusher member assembly axially translate with each other in unison to resheath the intravascular implant, while at least in a partially expanded deployed configuration, back into its compressed delivery configuration within the inner sheath lumen.
4. The intravascular implant delivery system of any of clauses 1-3, wherein the intravascular implant is one of stent, a stent graft, a flow-diverter, a vaso-occlusive device, and a vena cava filter.
5. The intravascular implant delivery system of any of clauses 1-4, wherein the pusher member assembly further comprises a proximal bumper and a distal bumper affixed to the pusher member, thereby forming an annular space therebetween, wherein the friction pad is disposed within the annular space.
6. The intravascular implant delivery system of any of clauses 1-5, wherein the pusher member comprises a delivery wire.
7. The intravascular implant delivery system of any of clauses 1-6, wherein the elastomeric base material of the friction pad is polyether block amide (PEBA).
8. The intravascular implant delivery system of any of clauses 1-7, wherein the at least one modulating element increases a ratio of a columnar strength over a radial strength of the tubular pad body.
9. The intravascular implant delivery system of any of clauses 1-8, wherein each of the at least one modulating element is elongated.
10. The intravascular implant delivery system of clause 9, wherein each of the at least one elongated modulating element extends parallel to a longitudinal axis of the tubular pad body.
11. The intravascular implant delivery system of any of clauses 1-10, wherein each of the at least one modulating element has a modulus of elasticity greater than a modulus of elasticity of the elastomeric base material.
12. The intravascular implant delivery system of clause 11, wherein each of the at least one modulating element is a solid beam.
13. The intravascular implant delivery system of clause 11, wherein the at least one modulating element increases a columnar strength of the tubular pad body.
14. The intravascular implant delivery system of clause 11, wherein the elastomeric base material has a durometer in the range of 25A-35D.
15. The intravascular implant delivery system of any of clauses 1-10, wherein each of the at least one modulating element has a modulus of elasticity less than a modulus of elasticity of the elastomeric base material.
16. The intravascular implant delivery system of clause 15, wherein each of the at least one modulating element is a gel or air.
17. The intravascular implant delivery system of clause 15, wherein the at least one modulating element decreases a radial strength of the tubular pad body.
18. The intravascular implant delivery system of clause 15, wherein the elastomeric base material has a durometer greater than 35D.
19. The intravascular implant delivery system of any of clauses 1-18, wherein the at least one modulating element comprises a plurality of modulating elements.
20. The intravascular implant delivery system of clause 19, wherein the plurality of modulating elements is circumferentially arranged within the elastomeric base material.
21. The intravascular implant delivery system of any of clauses 1-20, wherein the tubular pad body is cylindrical.
22. An intravascular implant delivery system, comprising: an intravascular implant having a compressed delivery configuration and an expanded deployed configuration, the intravascular implant comprising a tubular implant body and a central implant lumen axially extending through the tubular implant body; a delivery catheter having an elongate sheath body and an inner sheath lumen axially extending through the sheath body, the intravascular implant being disposed within the inner sheath lumen when in the compressed delivery configuration; and a pusher member assembly slidably disposed in the inner sheath lumen, the pusher member assembly comprising an elongate pusher member and a friction pad having a radially compressed state and a radially expanded state, the friction pad being disposed within the central implant lumen when in the radially compressed state, the friction pad comprising a tubular pad body and a central pad lumen axially extending through the tubular pad body, the pusher member disposed within the central pad lumen, the tubular pad body having an elastomeric base material that forms the tubular pad body and at least one modulating element embedded within the elastomeric base material, wherein the at least one modulating element increases a ratio of a columnar strength over a radial strength of the tubular pad body.
23. The intravascular implant delivery system of clause 22, wherein the pusher member assembly and delivery catheter can be axially translated relative to each other, and the friction pad is configured for frictionally engaging the intravascular implant as the pusher member assembly and delivery catheter are axially translated relative to each other, such that the intravascular implant and the pusher member assembly axially translate with each other in unison to deploy the intravascular implant from the inner sheath lumen into its expanded deployed configuration.
24. The intravascular implant delivery system of clause 23, wherein the friction pad is configured for frictionally engaging the intravascular implant as the pusher member assembly and the delivery catheter are axially translated relative to each other, such that the intravascular implant and the pusher member assembly axially translate with each other in unison to resheath the intravascular implant, while at least in a partially expanded deployed configuration, back into its compressed delivery configuration within the inner sheath lumen.
25. The intravascular implant delivery system of clause 22, wherein the intravascular implant is one of stent, a stent graft, a flow-diverter, a vaso-occlusive device, and a vena cava filter.
26. The intravascular implant delivery system of clause 22, wherein the pusher member assembly further comprises a proximal bumper and a distal bumper affixed to the pusher member, thereby forming an annular space therebetween, wherein the friction pad is disposed within the annular space.
27. The intravascular implant delivery system of clause 22, wherein the pusher member is a delivery wire.
28. The intravascular implant delivery system of clause 22, wherein the elastomeric base material of the friction pad is polyether block amide (PEBA).
29. The intravascular implant delivery system of clause 22, wherein each of the at least one modulating element is elongated.
30. The intravascular implant delivery system of clause 29, wherein each of the at least one elongated modulating element extends parallel to a longitudinal axis of the tubular pad body.
31. The intravascular implant delivery system of clause 22, wherein each of the at least one modulating element has a modulus of elasticity greater than a modulus of elasticity of the elastomeric base material.
32. The intravascular implant delivery system of clause 31, wherein each of the at least one modulating element is a solid beam.
33. The intravascular implant delivery system of clause 31, wherein the at least one modulating element increases a columnar strength of the tubular pad body.
34. The intravascular implant delivery system of clause 31, wherein the elastomeric base material has a durometer in the range of 25A-35D.
35. The intravascular implant delivery system of clause 22, wherein each of the at least one modulating element has a modulus of elasticity less than a modulus of elasticity of the elastomeric base material.
36. The intravascular implant delivery system of clause 35, wherein each of the at least one modulating element is a gel or air.
37. The intravascular implant delivery system of clause 35, wherein the at least one modulating element decreases a radial strength of the tubular pad body.
38. The intravascular implant delivery system of clause 35, wherein the elastomeric base material has a durometer greater than 35D.
39. The intravascular implant delivery system of clause 22, wherein the at least one modulating element comprises a plurality of modulating elements.
40. The intravascular implant delivery system of clause 39, wherein the plurality of modulating elements is circumferentially arranged within the elastomeric base material.
41. The intravascular implant delivery system of clause 22, wherein the tubular pad body is cylindrical.
42. An intravascular implant delivery system, comprising: an intravascular implant having a compressed delivery configuration and an expanded deployed configuration, the intravascular implant comprising a tubular implant body and a central implant lumen axially extending through the tubular implant body; a delivery catheter having an elongate sheath body and an inner sheath lumen axially extending through the sheath body, the intravascular implant being disposed within the inner sheath lumen when in the compressed delivery configuration; and a pusher member assembly slidably disposed in the inner sheath lumen, the pusher member assembly comprising an elongate pusher member and a friction pad having a radially compressed state and a radially expanded state, the friction pad being disposed within the central implant lumen when in the radially compressed state, the friction pad comprising a tubular pad body and a central pad lumen axially extending through the tubular pad body, the pusher member disposed within the central pad lumen, the tubular pad body having an elastomeric base material that forms the tubular pad body and at least one modulating element embedded within the elastomeric base material, wherein the at least one modulating element increases a columnar strength of the tubular pad body.
43. The intravascular implant delivery system of clause 42, wherein the pusher member assembly and delivery catheter can be axially translated relative to each other, and the friction pad is configured for frictionally engaging the intravascular implant as the pusher member assembly and delivery catheter are axially translated relative to each other, such that the intravascular implant and the pusher member assembly axially translate with each other in unison to deploy the intravascular implant from the inner sheath lumen into its expanded deployed configuration.
44. The intravascular implant delivery system of clause 43, wherein the friction pad is configured for frictionally engaging the intravascular implant as the pusher member assembly and the delivery catheter are axially translated relative to each other, such that the intravascular implant and the pusher member assembly axially translate with each other in unison to resheath the intravascular implant, while at least in a partially expanded deployed configuration, back into its compressed delivery configuration within the inner sheath lumen.
45. The intravascular implant delivery system of clause 42, wherein the intravascular implant is one of stent, a stent graft, a flow-diverter, a vaso-occlusive device, and a vena cava filter.
46. The intravascular implant delivery system of clause 42, wherein the pusher member assembly further comprises a proximal bumper and a distal bumper affixed to the pusher member, thereby forming an annular space therebetween, wherein the friction pad is disposed within the annular space.
47. The intravascular implant delivery system of clause 42, wherein the pusher member is a delivery wire.
48. The intravascular implant delivery system of clause 42, wherein the elastomeric base material of the friction pad is polyether block amide (PEBA).
49. The intravascular implant delivery system of clause 42, wherein each of the at least one modulating element is elongated.
50. The intravascular implant delivery system of clause 49, wherein each of the at least one elongated modulating element extends parallel to a longitudinal axis of the tubular pad body.
51. The intravascular implant delivery system of clause 42, wherein each of the at least one modulating element has a modulus of elasticity greater than a modulus of elasticity of the elastomeric base material.
52. The intravascular implant delivery system of clause 51, wherein each of the at least one modulating element is a solid beam.
53. The intravascular implant delivery system of clause 51, wherein the at least one modulating element increases a columnar strength of the tubular pad body.
54. The intravascular implant delivery system of clause 51, wherein the elastomeric base material has a durometer in the range of 25A-35D.
55. The intravascular implant delivery system of clause 42, wherein the at least one modulating element comprises a plurality of modulating elements.
56. The intravascular implant delivery system of clause 55, wherein the plurality of modulating elements is circumferentially arranged within the elastomeric base material.
57. The intravascular implant delivery system of clause 42, wherein the tubular pad body is cylindrical.
58. An intravascular implant delivery system, comprising: an intravascular implant having a compressed delivery configuration and an expanded deployed configuration, the intravascular implant comprising a tubular implant body and a central implant lumen axially extending through the tubular implant body; a delivery catheter having an elongate sheath body and an inner sheath lumen axially extending through the sheath body, the intravascular implant being disposed within the inner sheath lumen when in the compressed delivery configuration; and a pusher member assembly slidably disposed in the inner sheath lumen, the pusher member assembly comprising an elongate pusher member and a friction pad having a radially compressed state and a radially expanded state, the friction pad being disposed within the central implant lumen when in the radially compressed state, the friction pad comprising a tubular pad body and a central pad lumen axially extending through the tubular pad body, the pusher member disposed within the central pad lumen, the tubular pad body having an elastomeric base material that forms the tubular pad body and at least one modulating element embedded within the elastomeric base material, wherein the at least one modulating element decreases a radial strength of the tubular pad body.
59. The intravascular implant delivery system of clause 58, wherein the pusher member assembly and delivery catheter can be axially translated relative to each other, and the friction pad is configured for frictionally engaging the intravascular implant as the pusher member assembly and delivery catheter are axially translated relative to each other, such that the intravascular implant and the pusher member assembly axially translate with each other in unison to deploy the intravascular implant from the inner sheath lumen into its expanded deployed configuration.
60. The intravascular implant delivery system of clause 59, wherein the friction pad is configured for frictionally engaging the intravascular implant as the pusher member assembly and the delivery catheter are axially translated relative to each other, such that the intravascular implant and the pusher member assembly axially translate with each other in unison to resheath the intravascular implant, while at least in a partially expanded deployed configuration, back into its compressed delivery configuration within the inner sheath lumen.
61. The intravascular implant delivery system of clause 58, wherein the intravascular implant is one of stent, a stent graft, a flow-diverter, a vaso-occlusive device, and a vena cava filter.
62. The intravascular implant delivery system of clause 58, wherein the pusher member assembly further comprises a proximal bumper and a distal bumper affixed to the pusher member, thereby forming an annular space therebetween, wherein the friction pad is disposed within the annular space.
63. The intravascular implant delivery system of clause 58, wherein the pusher member is a delivery wire.
64. The intravascular implant delivery system of clause 58, wherein the elastomeric base material of the friction pad is polyether block amide (PEBA).
65. The intravascular implant delivery system of clause 58, wherein each of the at least one modulating element is elongated.
66. The intravascular implant delivery system of clause 65, wherein each of the at least one elongated modulating element extends parallel to a longitudinal axis of the tubular pad body.
67. The intravascular implant delivery system of clause 58, wherein each of the at least one modulating element has a modulus of elasticity less than a modulus of elasticity of the elastomeric base material.
68. The intravascular implant delivery system of clause 67, wherein each of the at least one modulating element is a gel or air.
69. The intravascular implant delivery system of clause 67, wherein the at least one modulating element decreases a radial strength of the tubular pad body.
70. The intravascular implant delivery system of clause 67, wherein the elastomeric base material has a durometer greater than 35D.
71. The intravascular implant delivery system of clause 58, wherein the at least one modulating element comprises a plurality of modulating elements.
72. The intravascular implant delivery system of clause 71, wherein the plurality of modulating elements is circumferentially arranged within the elastomeric base material.
73. The intravascular implant delivery system of clause 58, wherein the tubular pad body is cylindrical.

## Claims

1. An intravascular implant delivery system (100), comprising:
an intravascular implant (102) having a compressed delivery configuration and an expanded deployed configuration, the intravascular implant (102) comprising a tubular implant body (108) and a central implant lumen (114) axially extending through the tubular implant body (108);
a delivery catheter (104) having an elongate sheath body (116) and an inner sheath lumen (122) axially extending through the sheath body (116), the intravascular implant (102) being disposed within the inner sheath lumen (122) when in the compressed delivery configuration; and
a pusher member assembly (106) slidably disposed in the inner sheath lumen (122), the pusher member assembly (106) comprising an elongate pusher member (138);
the intravascular implant delivery system (100) **characterized in that** the pusher member assembly (106) further comprises a friction pad (162) having a radially compressed state and a radially expanded state, the friction pad (162) being disposed within the central implant lumen (114) when in the radially compressed state, the friction pad (162) comprising a tubular pad body (164) and a central pad lumen axially extending through the tubular pad body (164), the pusher member (138) disposed within the central pad lumen, the tubular pad body (164) having an elastomeric base material (184) that forms the tubular pad body (164) and at least one modulating element (186) embedded within the elastomeric base material (184), wherein the at least one modulating element (186) increases a ratio of a columnar strength over a radial strength of the tubular pad body (164).

2. The intravascular implant delivery system (100) of claim 1, wherein the pusher member assembly (106) and delivery catheter (104) can be axially translated relative to each other, and the friction pad (162) is configured for frictionally engaging the intravascular implant (102) as the pusher member assembly (106) and delivery catheter (104) are axially translated relative to each other, such that the intravascular implant (102) and the pusher member assembly (106) axially translate with each other in unison to deploy the intravascular implant (102) from the inner sheath lumen (122) into its expanded deployed configuration.

3. The intravascular implant delivery system (100) of claim 2, wherein the friction pad (162) is configured for frictionally engaging the intravascular implant (102) as the pusher member assembly (106) and the delivery catheter (104) are axially translated relative to each other, such that the intravascular implant (102) and the pusher member assembly (106) axially translate with each other in unison to resheath the intravascular implant (102), while at least in a partially expanded deployed configuration, back into its compressed delivery configuration within the inner sheath lumen (122).

4. The intravascular implant delivery system (100) of any of claims 1 - 3, wherein each of the at least one modulating element (186) is elongated.

5. The intravascular implant delivery system (100) of claim 4, wherein each of the at least one elongated modulating element (186) extends parallel to a longitudinal axis of the tubular pad body (164).

6. The intravascular implant delivery system (100) of any of claims 1 - 5, wherein each of the at least one modulating element (186) has a modulus of elasticity greater than a modulus of elasticity of the elastomeric base material (184).

7. The intravascular implant delivery system (100) of claim 6, wherein each of the at least one modulating element is a solid beam.

8. The intravascular implant delivery system (100) of claim 6, wherein the at least one modulating element increases a columnar strength of the tubular pad body (164).

9. The intravascular implant delivery system (100) of claim 6, wherein the elastomeric base material (184) has a durometer in the range of 25A-35D.

10. The intravascular implant delivery system (100) of claims 1 - 5, wherein each of the at least one modulating element has a modulus of elasticity less than a modulus of elasticity of the elastomeric base material (184).

11. The intravascular implant delivery system (100) of claim 10, wherein each of the at least one modulating element is a gel or air.

12. The intravascular implant delivery system (100) of claim 10, wherein the at least one modulating element decreases a radial strength of the tubular pad body (164).

13. The intravascular implant delivery system (100) of claim 10, wherein the elastomeric base material (184) has a durometer greater than 35D.

14. The intravascular implant delivery system (100) of any of claims 1 - 13, wherein the at least one modulating element comprises a plurality of modulating elements.

15. The intravascular implant delivery system (100) of claim 14, wherein the plurality of modulating elements is circumferentially arranged within the elastomeric base material (184).
